# EUROPEAN PATENT SPECIFICATION

(11) **EP 2 067 497 B1**
(45) Date of publication and mention of the grant of the patent: **15.02.2017**
(21) Application number: 07122405.9
(22) Date of filing: 05.12.2007
(51) Int. Cl.: A61M 15/00, A61M 16/10, A61M 16/00, A61M 11/00

(54) **Methods, systems and computer-readable products for optimizing aerosol particle administration to the lungs**
Verfahren, Systeme und computerlesbare Produkte zur Optimierung der Zuführung von Aerosolteilchen zur Lunge
Procédés, systèmes et produits lisible par un ordinateur pour optimiser l'administration de particules d'aérosol aux poumons

(43) Date of publication of application: 10.06.2009
(73) Proprietor: Vectura GmbH, 82131 Gauting (DE)
(72) Inventor: Müllinger, Bernhard, 80634 München (DE); Kroneberg, Philipp, 82140 Olching (DE); Wencker, Marion, Dr., 60327 Frankfurt (DE)
(74) Representative: Summersell, Richard John

(56) References cited:
- EP-A1- 1 700 614
- WO-A-98/52633
- FR-A1- 2 783 431
- US-A- 4 106 503
- US-A- 5 813 397
- US-A- 5 906 202
- US-A1- 2003 098 022
- US-A1- 2004 107 961
- US-A1- 2005 217 666

## Description

The present invention is directed to methods, systems and computer-readable products for optimizing aerosol particle administration to the lungs. More specifically, the present invention relates to methods of tailoring inhaling maneuvers for aerosol particle administration using the subject's lung function data and to systems and computer-readable products carrying out such methods.

Of particular interest to the invention are pulmonary delivery techniques which involve the inhalation of a pharmaceutical formulation by the patient so that a drug or active agent within the formulation can reach the lungs. Pulmonary delivery techniques can be advantageous for certain respiratory diseases in that it allows selective delivery of optimal concentrations of pharmaceutical formulations to the airways while causing fewer side effects than systematic administration. Although pulmonary delivery techniques allow selective delivery, effective administration of inhaled formulations has been found to vary depending on the mode of inhalation, aerosol particle characteristics and characteristics of the patient. Since many parameters determining administration are patient-related, patients often receive training for the proper breathing maneuver which ensures accurate administration of the formulation. However, such training can be an inconvenience to the patient and/or the health professional. Also, many patients find it cumbersome to maintain the proper breathing maneuver over time and, thus, patient compliance has not been ideal. Therefore, it would be advantageous to optimize pulmonary delivery techniques by adjusting the aerosol particle administration parameters of the inhalation device to the subject's breathing capabilities, such that it would be no longer necessary for patients to concentrate on the proper breathing maneuvers. For example, US 2001/0037806 A1 describes a device for the controlled inhalation of therapeutic aerosols.

WO 98/52633 describes an inhalation system which allows for the adaption of aerosol parameters such as inhalation volume and inspiratory flow rate, but fails to disclose how the specific values for volume and/or flow rate are selected or determined.

EP 1 700 614 A1 also describes inhalation systems which allow for the adaption of aerosol parameters such as inhalation volume and inspiratory flow rate, and further states that the desired values for inhalation volume and inspiratory flow rate may preferably be adjusted based on a patient's lung function parameters; namely the patient's lung volume (where appropriate minus a safety margin) and the inspiratory flow rate which the patient can achieve or tolerate. However, EP 1 700 614 A1 is silent as to which specific values of the lung volume or inspiratory flow rate are applied, how these would be tested and evaluated, and how the values may be converted, or translated, into appropriate aerosol parameters of the inhalation device. For instance, there are different commonly known lung volumes (e.g. tidal volume, inspiratory and expiratory reserve volumes, residual volume) and lung capacities; and oftentimes the inspiratory flow rate value achievable by a patient (i.e. the maximum) differs from the value considered comfortable during continuous inhalations.

US 4106503 A describes a system for administering a metered dose of nebulised antigen at incrementally increasing doses to test the patient's airflow resistance before and after antigen administration and adjust the following dose in accordance. US 4106503 A does not disclose the adaption of aerosol parameters of the inhalation system based on the patient's pulmonary function, such as adapting e.g. the inhalation volume delivered by the inhalation system

US 20030098022 A1 describes an inhalations system comprising a nebuliser and a peak flow meter to measure pulmonary function parameters, which are stored and/or displayed on a health server for doctor and patient to see. An adjustment of the inhalation system based on those pulmonary function parameters is not specifically disclosed.

US 20050217666 A1 describes a nebulizer-ventilator-system with a controller and a method and of operating this nebulizer, wherein a dose of aerosolised medicament is delivered intermittently to a ventilator circuit in such a way that medicament delivery efficiency is increased. In order to achieve this, the aerosol may be synchronized with an inspiratory phase of the ventilator cycle (e.g. within the first 50 percent of the inspiration), an "air chaser" may be supplied following the aerosol, and aerosols with smaller particle sizes about 1 to 5 µm are preferred.

US 20040107961 A1 discloses an inhalation device with a controller that may control e.g. the timing of drug administration or change drug regiments in response to a changing medical condition of a patient. The controller may e.g. be used to adjust the dosage of an administered drug for a particular circumstance, such as a particular time of day, an event (such as an activity that will require a dosage modification), or detection of a physiological condition (such as an adverse drug reaction that requires reduction or cessation of drug administration). In order to control the timing of drug administration; e.g. trigger the release of a dose at the onset of inhalation, the inhalation device comprises a sensor to detect the inherent pressure drop during inhalation. US 20040107961 A1 further suggests the use of clinical parameters such as blood oxygenation received by a portable pulse oximeter in order to alter a dosage regimen.

FR 2783431 A1 discloses a breath-actuated inhaler, which - based on flow rate/breath detection - triggers the administration of aerosol within the inhalation, or certain fractions of the inhalation only. The determination of pulmonary function parameters of the patient in order to adapt aerosol parameters for the inhaler and thereby optimize aerosol particle deposition is not described.

It is an object of the present invention to provide improved methods of optimizing aerosol particle administration by adapting aerosol parameters using the subject's lung function data.

It is also an object of the present invention to provide systems and computer-readable products for autonomously carrying out the methods of the present invention.

It is also an object of the present invention to provide an inhalation system having aerosol parameters such as the inhalation volume or the inspiratory flow rate that can be adapted automatically depending on measurement of at least one lung function parameter indicative of a subject's inhalation or exhalation function to thereby tailor aerosol particle administration to the subject's breathing capabilities.

The present invention comprises a method for determining aerosol particle administration to the lungs. The method comprises deriving aerosol parameters for an inhalation system, such as the inhalation volume or the inspiratory flow rate, based on at least one measured pulmonary function parameter to thereby adapt the aerosol particle administration to the subject's lung function. By adapting the aerosol particle administration to the subject's lung function, an optimal breathing pattern can be achieved. The user would no longer have to concentrate on the proper breathing technique because the present invention would induce the appropriate breathing pattern. This would inevitably improve user compliance and enable a precise, reproducible dosage of the pharmaceutical formulation to be delivered. The method may also comprise measuring at least one pulmonary function parameter of a subject.

In the prior art, the measured pulmonary function parameter is a measure of the subject's inspiratory function. Preferably, the inspiratory capacity (IC) of the subject is obtained. The inspiratory capacity is a measure of the maximal volume that the subject is capable of inhaling at a time. Using the subject's inhalation capacity, the appropriate inhalation volume and optimal aerosol parameters can be determined for the inhalation device or system. Preferably, the inhalation capacity is multiplied by a constant for determining the corresponding inhalation volume.

The inventors have unexpectedly found that, instead of requiring the subject's inspiratory capacity, expiratory function data can be used to sufficiently estimate the inspiratory capacity. As a measure of expiratory function, the pulmonary function parameter can be, for example, maximum expiratory flow MEF, forced expiratory flow FEF, forced expiratory vital capacity FVC or forced expiratory volume FEV, preferably forced expiratory volume per second FEV₁.

Preferably, the measured expiratory function is the forced expiratory volume per sec (FEV₁). This parameter describes the maximal volume that a patient is capable of exhaling in one second. The inventors have found that the FEV₁ can be used as a correlation of the IC if the FEV₁ is corrected to consider the severity grade of the presence of a lung disease in the subject. Depending on the severity grade of the subject, a corrective coefficient can be calculated. The measured FEV₁ can be multiplied by a corrective coefficient to obtain a corrected FEV₁ parameter. The corrected FEV₁ parameter can then be used to determine the appropriate inhalation volume and aerosol parameters. This aspect of the invention has several advantages. Although inspiratory capacity may be used for estimating inhalation volumes, inspiratory capacity calculations are in many cases prone to error. FEV₁ measurements, on the other hand, are generally quite reliable and reproducible. Further, FEV₁ measurements are common and can be obtained even with hand-held spirometers.

With the method of the present invention, the aerosol parameters can be adjusted to provide an optimal breathing pattern based on the subject's lung function data. The aerosol parameters include, but are not limited to, inhalation volume, the period between the number of breaths, inspiratory flow rate, and the timing of the release of aerosol particles (or aerosol bolus) and/or of particle-free air.

Aerosol parameter groups can be specified. Each aerosol parameter group can have predefined aerosol parameters. For example, each aerosol parameter group can have a different inhalation volume and/or different values for the aerosol parameters, such as aerosol bolus timing and inspiratory flow rate for example. The aerosol parameter group data can be stored in a memory means of the inhalation device or system, in a computer-readable medium, in an external storage device and/or transferred electronically to the inhalation device or system. In this aspect of the invention, the subject's lung function data is used to select the aerosol parameter group that enables the best breathing maneuver for the subject.

When the measured pulmonary function is a measure of the subject's inspiratory function, each aerosol parameter group has predetermined ranges for the measured inspiratory function parameter. Preferably, the aerosol parameters can be derived by comparing the value of the measured inspiratory function with predetermined ranges for prespecified aerosol parameter groups and by selecting the aerosol parameter group having a range, within which the measured inspiratory function parameter falls. Preferably, the measured inspiratory function parameter is the inspiratory capacity.

In the invention, when the pulmonary function parameter is a measure of expiratory function, each aerosol parameter group has predetermined ranges for the corrected expiratory function. For example, the predetermined range values could be the same as those used for the case where inspiratory function data is considered. Preferably, the aerosol parameters are derived by selecting a corrective coefficient using the measured expiratory function from among a plurality of corrective coefficients derived from a standard, multiplying the measured expiratory function by the corrective coefficient to obtain a corrected pulmonary function parameter value, comparing the corrected pulmonary function parameter value with the predetermined ranges for the aerosol parameter groups, and selecting the aerosol parameter group having a range, within which the corrected pulmonary function parameter value falls.

The present invention is also directed to a computer readable medium comprising encoding means configured to implement the methods of the present invention. Preferably, the methods of the present invention are computer-implemented methods that can be carried out by a processor or controller of the inhalation device or system and/or by an external device.

The present invention is also directed to an inhalation system for administering aerosol particles to the lungs comprising a controller for adapting aerosol parameters for said inhalation system based on at least one measured pulmonary function parameter. Preferably, the controller comprises a processor and encoding means causing the system to implement the methods of the present invention. The system of the present invention can be designed to adapt the subject's inhalation maneuver automatically depending on the subject's lung function data. The system may also record and store the subject's breathing maneuvers, e.g., for compliance control. The system may also comprise a monitor for measuring at least one pulmonary function parameter of a subject.

The system of the present invention may be in any suitable form for an inhalation system. For example, the system of the present invention may be designed to receive a variety of detachable components, such as a mouthpiece, nebulizer or the like, and at least one cartridge or the like containing the pharmaceutical formulation. For example, the system of the present invention may comprise a mouthpiece connected in fluid communication with the inhalation flow path. The mouthpiece may be a permanent part of the housing or a detachable part.

Preferably, the system of the present invention comprises at least one orifice connectable to a source of aerosolized particles. The aerosolized particle source is preferably releasably or detachably connected to the device by any suitable means known in the art. The aerosolized particle source may be a powder dispersion device which utilizes a compressed gas to aerosolize a powder. The aerosolized particle source may be a nebulizer or the like, for aerosolizing solid or liquid particles. The nebulizer may be an ultrasonic nebulizer, a vibrating mesh nebulizer, a jet nebulizer or any other suitable nebulizer or vaporizer known in the field. These nebulizers can be separate components which can be attached to the system before use.

The system of the present invention may also comprise a reader for reading a memory means having a subject's individual parameters, lung function data and/or aerosol depositing parameters stored thereon. The memory means can be in the form of any computer readable storage medium known in the art, such as but limited to a storage stick, memory disk or electronic data card, such as a smart card. The reader can be in any form as known in the art. For example, the reader can be an interface or port, e.g. a USB port or the like for receiving a storage stick or a serial connection or drive for receiving a memory or electronic data card.

The system of the present invention may also comprise at least one communication means for receiving and/or sending data associated with a subject's individual parameters, lung function data and/or aerosol depositing parameters. The communication means may be a wired connection or wireless connection sending and/or receiving data via infrared, microwave or radio frequency, optical techniques or any suitable manner known in the art. The communication means may be a telephone connection or jack. This would be advantageous if a health professional, e.g. a doctor, would like to adjust, from a remote location, the aerosol parameters based on lung function.

One object of the present invention is to provide an inhalation system having aerosol parameters, such as the inhalation volume or the inspiratory flow rate, that can be adapted' automatically depending on measurement of at least one lung function parameter indicative of a subject's inhalation function or preferably exhalation function to thereby tailor aerosol particle administration to the subject's breathing capabilities. Some examples of inhalation systems which can be used in conjunction with the methods and products of the present invention are described in WO 98/52633, EP 1 700 614 A1, European patent application no. 07 113 705.3 and European patent application no. 07 115 812.5.

The "aerosol particles" administered by the present invention preferably comprise at least one pharmaceutical formulation. The pharmaceutical formulations that may be aerosolized include powdered medicaments, liquid solutions or suspensions and the like and may include an active agent.

The term "pharmaceutical formulation" as used herein, includes active ingredients, drugs, medicaments, compounds, compositions, or mixtures of substances bringing about a pharmacological, often advantageous, effect. It includes food, food supplements, nutrients, medicaments, vaccines, vitamins, and other useful active ingredients. Moreover, the terms, as used herein, include any physiologically or pharmacologically active substances, bringing about a topical or systemic effect in a patient. The active ingredient lending itself to administration in the form of an aerosol can be an antibody, antiviral active ingredient, anti-epileptic, analgesic, anti-inflammatory active ingredient, and bronchodilator or can be an organic or inorganic compound, which without any restrictions can also be a medicament having an effect on the peripheral nervous system, adrenergic receptors, cholinergic receptors, skeletal muscles, cardiovascular system, unstriated muscles, circulatory system, neuronal connections, pulmonary system, respiratory system, endocrine and hormonic system, immune system, reproductive system, skeletal system, food supply system and excretory system, histamine cascade or central nervous system. Suitable active ingredients are for instance polysaccharides, steroids, hypnotics and sedatives, activators, tranquilizers, anticonvulsives (antispasmodics) and muscle relaxants, anti-Parkinson-substances, analgesics, anti-inflammatory agents, antimicrobial active ingredients, antimalarial agents, hormones, including contraceptives, symphatocomimetics, polypeptides and proteins producing physiological effects, diuretics, substances regulating the lipometabolism, anti-androgenic active ingredients, antiparasitics, neoplastic and antineoplastic agents, antidiabetics, food and food supplements, growth-promoters, fats, stool-regulators, electrolytes, vaccines and diagnostics.

The invention is particularly suited for inhalation application of different active ingredients, such as the following ones (without being restricted thereto): Insulin, calcitonin, erythropoietin (EPO), factor VII, factor IX, cylcosporin, granulozyte colony stimulating factor (GCSF), alpha-1-proteinase inhibitor, elcatonin, granulocyte macrophage colony stimulating factor (GMCSF), growth hormones, human growth hormone (HGH), growth hormone releasing hormone (GHRH), heparin, low molecular weight heparin (LMWH), interferon alpha, interferon beta, interferon gamma, interleukin-2, luteinizing hormone releasing hormone (LHRH), somatostatin, somatostatin-analogs; including octreotides, vasopressin analogs, follicle stimulating hormone (FSH), insulin-like growth factor, insulintropin, interleukin-I receptor antagonist, interleukin-3, interleukin-4, interleukin-6, macrophage colony stimulating factor (M-CSF), nerve growth factor, parathryoid hormone (PTH), thymosin alpha 1, IIb/IIIa inhibitor, alpha-1 antitrypsin, antibodies against respiratorily syncytic virus, cystic fibrosis transmembrane regulator gene (CFTR), desoxyribonuclease (Dnase), bactericides, permeability increasing protein (BPI), anti-CMV antibodies, interleukin-1-receptor, retinol, retinyl-ester, tocopherols and their esters, tocotrienols and their esters, carotinoids, in particular beta carotin and other natural and synthetic antioxidants, retinol acids, pentamides, albuterolsulfate, metaproterenolsulfate, beclomethasonedipropionate, triamcinolonacetamide, budesonidacetonides, ipratropium bromide, salbutamols, formanilides, flunisolides, fluticasones, cromolyn potassium, ergotamine tartrate and the analogs, agonists and antagonists of the above-mentioned substances.

Moreover, active ingredients can be nucleic acids in the form of pure nucleic acid molecules, viral vectors, associated viral particles, nucleic acids associated with or contained in lipids or a lipid-containing material, plasmid DNA or plasmid RNA or other constructs from nucleic acids, which are suitable for cell transfection or cell transformation, in particular in the case of cells of the alveolar region of the lung.

The active ingredient can be present in different forms, such as soluble or insoluble, charged or uncharged molecules, components of molecular complexes or pharmacologically acceptable inactive ingredients. The active ingredient can consist of naturally occurring molecules or their recombinant products, or the molecules can be analogs of the naturally occurring or recombinantly produced active ingredients to which or from which one or more amino acids have been added or deleted. Moreover, the active ingredient can contain attenuated live vaccines or killed viruses for vaccination purposes. If the active ingredient is insulin, it includes naturally extracted human insulin, recombinant human insulin, insulin extracted from cattle and/or swine, recombinant porcine or bovine insulin and mixtures of the above-mentioned insulins. The insulin can be present in a substantially purified form, but can also contain usual commercial extracts. The term "insulin" also includes analogs, to which or from which one or more amino acids of the naturally occurring or recombinant insulin have been added or deleted.

In an experimental study, the methods of the present invention were used to determine aerosol parameters for patients diagnosed with a lung disease, like chronic obstructive pulmonary disease (COPD) or asthma. The patient's lung function data was evaluated and used for allocating the patient to the best-suited aerosol parameter group. In this case, four aerosol parameter groups were set up, wherein each group was associated with a certain aerosol parameter, e.g. the inhalation volume. One of the purposes of the experimental study was to compare aerosol parameter group allocation achieved using inspiratory capacity data with aerosol parameter allocation achieved using forced expiratory volume data.

In a first aspect of the study, each patient was allocated to an aerosol parameter group depending on the patient's inhalation capacity (IC) data. In the particular cases, the IC ranges used for the aerosol parameter groups are shown below in Table 1.

**Table 1**

| **IC in [1]** | **Group** | **Inhalation Volume in [1]** |
|---|---|---|
| 0.0 - 0.75 | excluded | 0.0 |
| 0.76 - 1.53 | 1 | 0.5 |
| 1.54 - 2.30 | 2 | 1.0 |
| 2.31 - 3.10 | 3 | 1.5 |
| higher than 3.10 | 4 | 2.0 |

The aerosol parameter group was determined by comparing the patient's IC with the preset IC ranges associated with the groups and selecting the group having a range, within which the patient's IC fits. For example, one patient in the study had an inhalation capacity of approximately 1.781. For that patient, the aerosol parameter group 2 was selected.

In another aspect of the invention, each patient was allocated to an aerosol parameter group depending on the patient's forced expiratory volume per sec (FEV₁) data. The FEV₁ measurements were corrected using a corrective coefficient that was determined using the severity grade of the patient's lung disease, i.e. lung diseases like COPD or asthma GOLD standard.

The patients were classified into different categories depending on the severity of the disease. The classification for COPD i.e. is standard regulated by the Global initiative for chronic Obstructive Lung Disease and is commonly referred to as the GOLD class or standard. In the GOLD class, the classification of severity of obstructive lung disease presently includes four stages determined by spirometry measurements: stage I (mild) for FEV₁≥80% predicted (using appropriated normal values for the person's gender, age and height), stage II (moderate) for 50% ≤ FEV₁≤80% predicted; stage III (severe) for 30%≤ FEV₁≤50% predicted; and stage IV (very severe) for FEV₁≤30% predicted. For each GOLD class, a corrective coefficient was calculated. These values are shown below in Table 2. FEV1_{gc} represents the corrected FEV₁ measurement. It should be noted that the various values for the GOLD classes can change yearly as they depend at least in part on experimental data.

**Table 2**

| **Severity Grade (Gold class)** | **FEV1 % of predicted** | **Corrective Coefficient (k)** | **Correction of FEV₁** |
|---|---|---|---|
| 1 | > 80% | k1 ≈ 1.10 | FEV1_{gc} = FEV₁*k1 |
| 2 | 50% - 80% | k2 ≈ 1.29 | FEV1_{gc} = FEV₁*k2 |
| 3 | 30% - 50% | k3 ≈ 1.60 | FEV1_{gc} = FEV₁*k3 |
| 4 | < 30% | k4 ≈ 2.42 | FEV1_{gc} = FEV₁*k4 |

Referring again to the patient with an inhalation capacity of 1.781, the patient had a measured FEV₁ of 1.471 which was 88% of the predicted value considering the patient's age, height and gender. As can be seen from Table 2, the patient would be classified in GOLD class 1. The GOLD class can be determined based on the measured FEV₁. The FEV1_{gc} parameter is obtained by multiplying the measured FEV₁ with the corrective coefficient. The corrective coefficient for GOLD class 1 is 1.10. In this case, FEV₁ x k1 (1.47 x 1.10) yielded a FEV1_{gc} parameter of 1.62. As can be taken from Table 3 shown below, the patient would again be allocated to group 2.

**Table 3**

| **FEV1_{gc} in [1]** | **Group** | **Inhalation Volume in [1]** |
|---|---|---|
| 0.0 - 0.75 | excluded | 0.0 |
| 0.76 - 1.53 | 1 | 0.5 |
| 1.54 - 2.30 | 2 | 1.0 |
| 2.31 - 3.10 | 3 | 1.5 |
| higher than 3.10 | 4 | 2.0 |

In the majority of the cases, the patients were allocated to the same aerosol parameter groups irrespective of whether the IC data or the FEV data was used. The experimental results. suggest that the FEV₁ data could be reliably used, in place of the IC data for determining aerosol parameters.

Some embodiments of the inhalation system of the invention will be described with reference to the following figures:
- **Figure 1**: is a schematic view of an embodiment of the system.
- **Figure 2**: is a schematic view of another embodiment of the system.
- **Figure 3**: is a schematic view of another embodiment of the system.
- **Figure 4**: is a perspective view of another embodiment of the system.

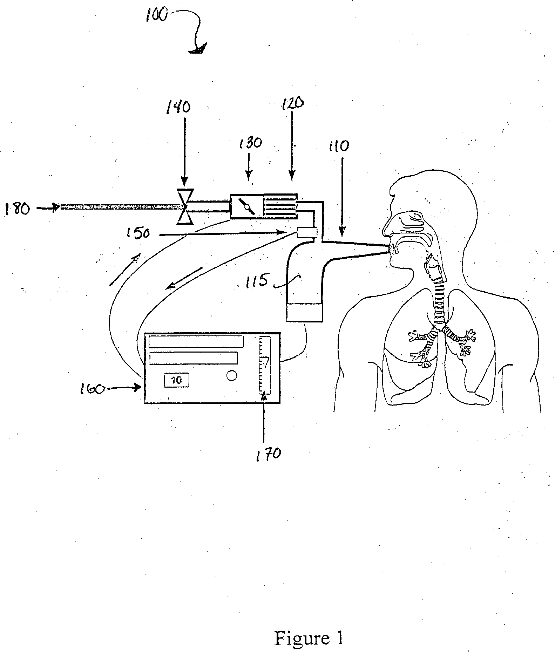

Figure 1 illustrates a schematic view of an embodiment of a system which can be used for carrying out the methods of the present invention. Such a prior art system is disclosed in WO 98/52633. System 100 for the administration of a medicated aerosol through the lung comprises an inhalation mouthpiece 110 with an associated vaporiser 115 which can be adjusted in terms of its operating phases as well as intensity/frequency. A volumetric flow controller 120, a compressed-air control valve 130, which is preferably configured as solenoid valve, a pressure reducer 140 and a compressed-air inlet 180 are disposed to be in flow communication with the inhalation mouthpiece 110. System 100 may also comprise a pressure sensor 150 which is responsive to a suction pressure in the mouthpiece for triggering the beginning of the vaporising operation of the vaporiser 115.

An electronic controller 160 is functionally connected to the compressed-air control valve 130, the pressure sensor 150 and the vaporiser 115. The electronic controller 160 is schematically represented as housing block which is additionally provided with an optical display of a flow meter 170 for checking the inhalation flow, for instance over a range of values from 0 to 1000 cm³/s. The volumetric flow controller 120 serves to maintain the inhalation flow constant over a particular range, for example from 0 to 1000 cm³/s. The compressed-air valve 130 is preferably designed as solenoid valve which switches the air supply.

Moreover, the inhalation period, the pause interval and the number of breathing cycles can be set on the electronic controller 160 in a manner not illustrated here, with a light-emitting diode being provided to issue a pause signal. For example, the individual functions which can be set, such as vaporising period or inhalation time, the pause interval and the ultrasonic vaporiser control, can be mentioned in text form in a block of the electronic controller 160, with a breathing cycle counter being included for detection of the breathing cycles.

In system 100, the nominal flow of inhalation can be initially controlled by means of flow meter 170, which includes a floating body, and then set to the desired amount at the volumetric flow controller 120 which maintains the inhalation flow constant. Then, the desired inhalation period can be set, e.g. using an input means of the electronic controller 160, within a particular range, for example from 0 to 20 seconds. The inhaled volume is then derived from the inhalation period and the inhalation flow. The desired duration of the pause interval can be equally set to be within a range, for example from 9 to 20 seconds. Additionally, the breathing cycle counter can be set to zero.

For instance, following these preparations, inhalation can now be performed in a way that the patient inhales at mouthpiece 110, which causes the pressure sensor 150 to respond and start inhalation automatically. The vaporiser 115 is supplied with compressed air throughout the pre-selected inhalation period, and the desired medicament is discharged in the form of a medicated aerosol from the mouthpiece 110 at a pre-selected flow rate. Upon expiration of the inhalation period, the compressed-air supply can be interrupted so that the subject cannot continue inhalation. The light-emitting diode signals to the subject that he or she should hold his or her breath. As soon as the pause interval has elapsed the pause interval LED can be extinguished, the patient exhales and the breathing cycle counter is incremented.

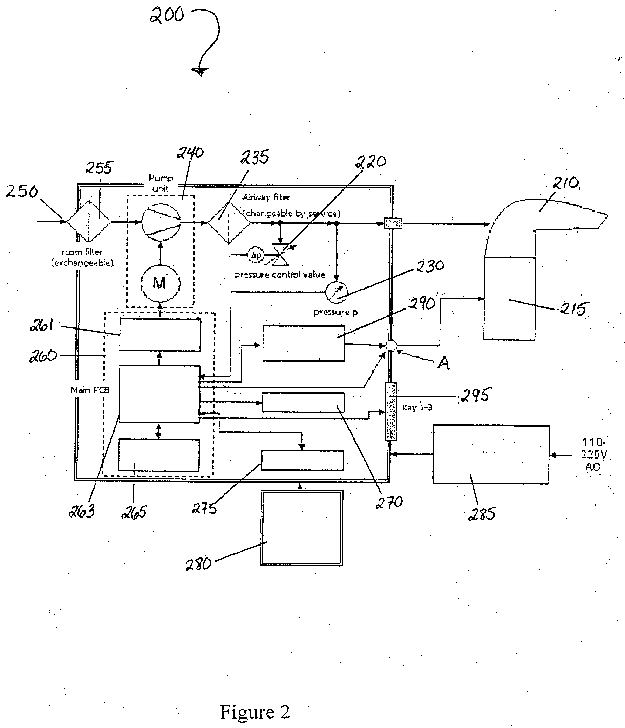

Figure 2 schematically illustrates another embodiment of a system which can be used with the methods of the present invention. Such a system is described in EP 1 700 614 A1. System 200 for the application of a pharmaceutical aerosol via the lung comprises an inhalation mouthpiece 210 with an associated nebulizer 215. A pressure control valve 220, a pressure sensor 230, an exchangeable filter 235 (or pressure attenuator), a (pressure) air pump 240, a further exchangeable filter 255, and an air inlet 250 are operably connected to the inhalation mouthpiece 210. The filter 255 is assigned to the air inlet 250 in order to filter the air supplied to the air pump 240. The filter 235, the pressure sensor 230 and the pressure control valve 220 are arranged downstream from the air pump 240. The pressure sensor 230 is responsive to suction pressure in the mouthpiece 210 to trigger the nebulization start of the nebulizer 215.

System 200 may comprise control unit 260 having a motor controller 261, a microprocessor 263 and a memory 265. The control unit 260 may be functionally connected to the pump 240, the pressure sensor 230 and to the nebulizer 215 via corresponding nebulizer electronics 290 (e.g., a printed circuit board). The nebulizer electronics are preferably exchangeable so that the corresponding electronics can be used for the respective nebulizers. The nebulizer is connectable to the inhalation component via a connecting point A. Preferably, the connection A is an encoded connection, which thus enables an automatic recognition of the connected nebulizer. Thus, a plausibility test can be carried out, e.g., whether the pharmaceutical to be administered can be applied with the connected nebulizer at all.

The pressure sensor 230 can be used as feedback to the control unit 260 and serves to keep the inhalation flow constant in a particular range, for example from 0 to 1000 cm³/s.

In the control unit 260, the pharmaceutical and the inhalation volume can be set or adjusted via an input unit 295, e.g., via a keyboard or individual input keys. System 200 may also comprise a SmartCard device reader 275 via which individual inhalation parameters such as inhalation flow and inhalation volume stored on a SmartCard device 280 can be read in. The individual parameters can be stored in the memory 265.

System 200 may also comprise an optical display unit 270 in order to show the subject the current inhalation flow. The display also serves to display the name of the pharmaceutical currently used, error messages, etc. The display preferably also serves to display the error messages of the nebulizer. System 200 may also comprise a power supply unit 285 (e.g., 110-240 V) for supplying current to the system.

The inhalation is carried out such that the patient inhales at the mouthpiece 210, whereby the pressure sensor 230 reacts and automatically starts the inhalation. The nebulizer 215 is supplied with compressed air via the air pump 240 during the inhalation, and the desired pharmaceutical in the form of aerosol emits with a pre-selected or predetermined flow from the mouthpiece 210. After expiry of the inhalation period and/or achievement of the inhalation volume, the air supply is interrupted and the patient cannot inhale further. The control unit 260 may control or activate the air pump 240 when the start of an inhalation process is recognized by the pressure sensor 230. When the inhalation process is terminated, the air pump 240 is deactivated again by the control unit 260.

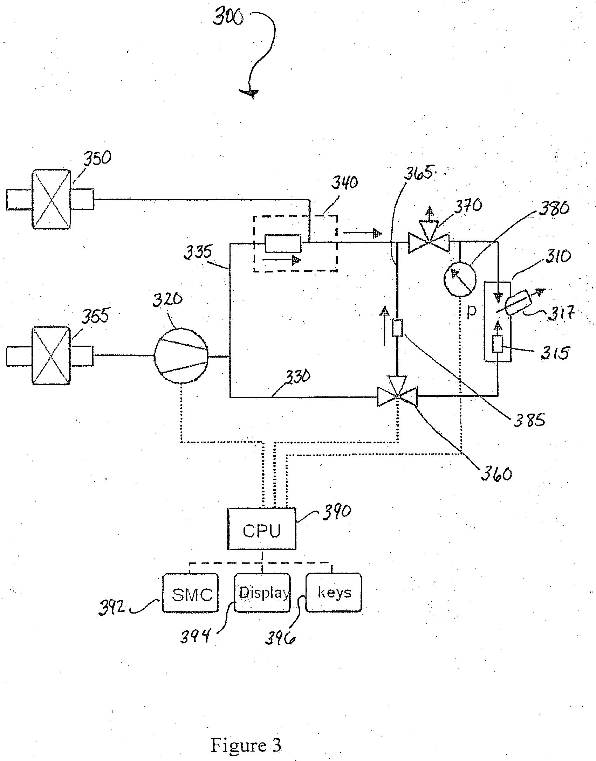

Figure 3 shows a schematic view of another embodiment of a system which can be used for the methods of the present invention. Such a system is disclosed in European patent application no. 07 115 815.5. System 300 comprises a compressor 320 for providing compressed air flow and a nebulizer 315 for providing aerosolized particle flow. The compressor 320 and the nebulizer 315 are connected to each other by means of an air channel 330. Further, system 300 comprises a mixer 310 for receiving the compressed air and/or the aerosolized particle flow and for providing at an outlet either a mixture of the compressed air flow and the aerosolized particle flow or only the compressed air flow or only the aerosolized particle flow. The mixer 310 may have an inhalation mouthpiece 317 provided at an outlet of the mixer 310. In a preferred embodiment of this system, the nebulizer 315 and mixer 310 are integrated as a single component as shown in Figure 3. However, it is also possible to provide the nebulizer 315 and mixer 310 as separate components. In this case, the nebulizer and mixer can be connected using a channel.

The mixer 310 of system 300 is connected to the compressor 320 by an air channel 335. In this respect, the compressed air flow leaving the compressor 320 is separated into different flow paths; e.g., one flowing through air channel 330 and one flowing through air channel 335, as shown in Figure 3.

System 300 may comprise a flow amplifier 340 as a means for increasing the compressed air flow. As shown in Figure 3, the flow amplifier can be placed in fluid communication with air channel 335. In this respect, the flow amplifier is in fluid communication with mixer 310. The flow amplifier 340 can be in the form of venturi injectors 340. The venturi injectors use additional air flow, which is provided through air filter 350. The compressor 320 may also have an air filter 355 located at an inlet. The use of a flow amplifier provides several advantages. For one, the size of the inhalation system can be significantly reduced because a smaller compressor can be used. Also, with the use of a flow amplifier, it is no longer necessary to have a separate pressure-relief valve.

System 300 may also comprise a by-pass channel 365 that can be opened or closed using a by-pass valve 360. The by-pass channel 365 enables a constant inhalation flow even when aerosolizing is shut on or off. For example, this may be the case when aerosolizing is deactivated during the inhalation process. If the aerosolizing is interrupted during the inhalation process, the by-pass valve 360 is opened to redirect the compressed air flowing in air channel 330 from compressor 320 to by-pass channel 365. In this respect, the by-pass channel can be used to circumvent air flowing to the nebulizer 315. The by-pass channel 365 can be provided with an auxiliary injector 385 which functions in a similar manner as the nebulizer injector in nebulizer 315. The compressed air leaving the by-pass channel 365 and air channel 335 flows to mixer 310 such that a constant inhalation flow is provided to the patient even if the nebulizer is deactivated during the inhalation process. The deactivation of the nebulizer during inhalation may be advantageous for targeting aerosolized particles to particular areas of the lungs.

System 300 may also comprise a valve 370 for enabling excess air to be removed from the system, for example during an inhalation pause.

Further, system 300 may comprise a control unit 390 for controlling and/or regulating various components of the system, e.g., the valves and/or nebulizer. The control unit 390 can be configured to control the various components of the system according to a subject's individual aerosol and/or inhalation parameters. A pressure sensor 380 can be used for providing feedback to the control unit 390. Preferably, the control unit 390 has a display 394 and input means, such as a keyboard, for entering information. System 300 may also comprise input means such as a reader 392 for reading an electronic storage means, such as a memory card (e.g. a smart card), a storage stick, storage disk or the like.

In Figure 3, an exemplary system of this embodiment is shown. However, modifications to the system shown in Figure 3 are possible and foreseen. For example, system 300 may comprise venturi injectors 340 without the presence of the by-pass channel 365 or may comprise a by-pass channel 365 without the presence of the venturi injectors 340.

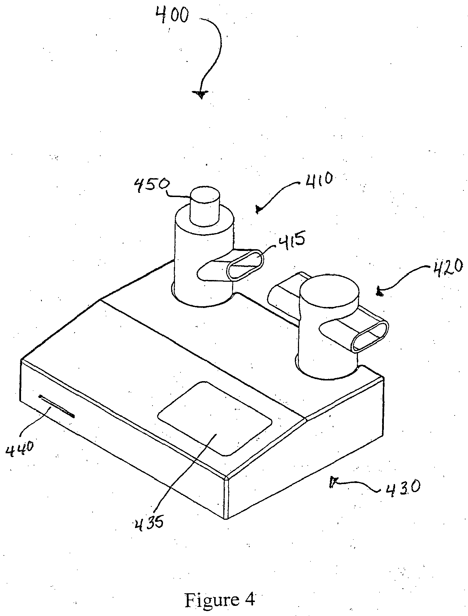

In Figure 4, another embodiment of an inhalation system which can be used with the methods of the present invention is illustrated. Such a system is disclosed in European patent application 07 113 705.3. In this embodiment, the system 400 comprises an inhalation device 410, a spirometer 420 for measuring a lung function parameter and a base station 430 for receiving the device 410 and/or spirometer 420.

As depicted, the device 410 can be a hand-held, portable device. The device 410 has a housing with a mouthpiece 415. The mouthpiece 415 may be removed or replaced by a compatible mouthpiece. To this end, a connection is provided in the housing of device 410 for enabling a detachable connection with the mouthpiece 415. Alternatively, mouthpiece 415 may be an integral part of the housing of the device 410. Device 410 is also adapted to receive a cartridge or receptacle 450 holding the pharmaceutical formulation or drug. For example, the housing of device 410 can be manufactured such that cartridge 450 can simply be inserted into the top of the device as shown in Figure 4.

The spirometer 420 can also be a hand-held, portable device as shown. Spirometer 420 may also have control buttons for controlling the operations of the spirometer and/or a display for showing measured results and/or settings.

The base station 430 may include cradles, or the like, for holding the device 410 and spirometer 420. The base station 430 may also serve as a charger for recharging any batteries provided in device 10 and/or spirometer 420. To this end, the cradles may include an interface enabling an electronic connection with device 410 or monitor 420. The interface could also enable the transfer of data between the base station 430 and the device 410 or spirometer 420. As depicted, base station 430 may also have a display 435 for displaying any desired information or data, for example the status of the base station 430, device 410 and/or spirometer 420. Base station 430 may optionally include a slot 440 for receiving a memory card, e.g. a smart card, having data with a subject's aerosol parameters. In this respect, multiple users could use the base station 430 for adapting their inhalation devices 410. Base station 430 may also include an additional reader for reading a storage medium like a memory stick. Although not shown, base station 430 may include communication means for enabling wired or wireless telecommunication and/or data transfer to and from a remote location.

In Figure 4, an exemplary system of this embodiment is shown. However, modifications to the illustrated system are possible and foreseen. For example, the base station 430 of system may be used for receiving an inhalation device having an integrated spirometer and an additional monitor for measuring a health parameter such as a cardio-monitor measuring heart beat rate for example. The inhalation device itself may also have communication means and/or a card reader, for example.

The various embodiments and experimental results presented in the specification are used for the sake of description and clarification of the invention, and thus should not be interpreted as limiting the scope of the invention as such. Moreover, the present invention is realized by the features of the claims and any obvious modifications thereof.

### LIST OF REFERENCE NUMERALS

- **100**: **system**
- 110: inhalation mouthpiece
- 115: vaporizer
- 120: volumetric flow controller
- 130: compressed-air control valve
- 140: pressure reducer
- 150: pressure sensor
- 160: electronic controller
- 170: flow meter
- 180: compressed-air inlet

- **200**: **system**
- 210: inhalation mouthpiece
- 215: nebulizer
- 220: pressure control valve
- 230: pressure sensor
- 235: exchangeable filter
- 240: airpump
- 250: air inlet
- 255: filter for air inlet
- 260: control unit
- 261: motor controller
- 263: microprocessor
- 265: memory
- 270: display
- 275: card reader
- 280: card device
- 285: power supply
- 290: nebulizer electronics
- 295: input unit
- A: connecting point

- **300**: **system**
- 310: mixer
- 315: nebulizer
- 317: inhalation mouthpiece
- 320: compressor
- 330: air channel
- 335: air channel
- 340: venturi injectors
- 350: filter
- 355: filter for compressor
- 360: by-pass valve
- 365: by-pass channel
- 370: valve
- 380: pressure sensor
- 385: auxiliary injector
- 390: control unit
- 392: reader
- 394: display
- 396: keyboard

- **400**: **system**
- 410: inhalation device
- 415: inhalation mouthpiece
- 420: spirometer
- 430: base station
- 435: display
- 440: slot for reader
- 450: cartridge

## Claims

1. A method for determining aerosol particle administration to the lungs, the method comprising:
deriving aerosol parameters for an inhalation system (100, 200, 300, 400) based on at least one measured pulmonary function parameter to thereby adapt the aerosol particle administration to the subject's lung function, **characterised in that**
the pulmonary function parameter is a measure of an expiratory function, and
the step of deriving aerosol parameters comprises:
selecting a corrective coefficient using said measured expiratory function from among a plurality of corrective coefficients derived from a standard;
multiplying said measured expiratory function by said corrective coefficient to obtain a corrected pulmonary function parameter value;
comparing said corrected pulmonary function parameter value with predetermined ranges for prespecified aerosol parameter groups, each aerosol parameter group having predefined aerosol parameters; and
selecting a prespecified aerosol parameter group based on said comparison.

2. The method of claim 1, further comprising measuring at least one pulmonary function parameter of a subject

3. The method of claim 1 or 2, wherein said corrective coefficient is based on the severity grade of the subject's lung disease.

4. The method of claim 1, 2, or 3, wherein said pulmonary function parameter is maximum expiratory flow MEF, forced expiratory flow FEF, forced expiratory vital capacity FVC or forced expiratory volume FEV, preferably forced expiratory volume per second FEV 1.

5. A computer readable medium (280) comprising encoding means implement configured to the method of any of claims 1 to 4.

6. An inhalation system (100, 200, 300, 400) for administering aerosol particles to the lungs comprising:
a controller (160, 260, 390) arranged to adapt aerosol parameters for said inhalation system based on at least one measured pulmonary function parameter, wherein said controller (160, 260, 390) comprises encoding means configured to cause the system to implement the method of any of claims 1 to 4.

7. The system of claim 6, wherein said system comprises a reader (275, 392) for reading the computer readable medium (280) of claim 5 or for reading a memory means having a subject's individual data and/or aerosol parameters stored thereon, and/or wherein said controller (160, 260, 390) has a memory for storing a subject's individual data and/or aerosol parameters.

8. The system of any of the claims 6 to 7, further comprising a monitor (420) for measuring at least one pulmonary function parameter of a subject.

9. The system of any of the claims 6 to 8, wherein said aerosol parameters comprise the inspiratory volume of aerosol particles and/or aerosol-free particles, the inspiratory flow rate and/or the timing of the inspiratory flow of aerosol particles and/or aerosol-free particles.

10. The system of any of the claims 6 to 9, wherein the system is a portable, hand-held inhalation device.

## Patentansprüche

1. Verfahren zur Bestimmung der Verabreichung von Aerosolpartikeln an die Lunge, umfassend:
Ableiten von Aerosolparametern für ein Inhalationssystem (100, 200, 300, 400) auf der Grundlage von mindestens einem gemessenen Lungenfunktionsparameter, um dadurch die Aerosolpartikelverabreichung an die Lungenfunktion des Probanden anzupassen, **dadurch gekennzeichnet, dass** der Lungenfunktionsparameter ein Maß einer Exspirationsfunktion ist und
der Schritt des Ableitens von Aerosolparametern Folgendes umfasst:
Wählen eines Korrekturkoeffizienten unter Verwendung der gemessenen Exspirationsfunktion aus einer Vielzahl von Korrekturkoeffizienten, die von einem Standard abgeleitet sind,
Multiplizieren der gemessenen Exspirationsfunktion mit dem Korrekturkoeffizienten, was einen korrigierten Lungenfunktionsparameterwert ergibt,
Vergleichen des korrigierten Lungenfunktionsparameterwerts mit vorbestimmten Bereichen für im Voraus spezifizierten Aerosolparametergruppen, wobei jede Aerosolparametergruppe vordefinierte Aerosolparameter hat, und
Wählen einer im Voraus spezifizierten Aerosolparametergruppe auf der Grundlage des Vergleichs.

2. Verfahren nach Anspruch 1, ferner umfassend Messen mindestens eines Lungenfunktionsparameters eines Probanden.

3. Verfahren nach Anspruch 1 oder 2, wobei der Korrekturkoeffizient auf dem Schweregrad der Erkrankung der Lunge des Probanden beruht.

4. Verfahren nach Anspruch 1, 2 oder 3, wobei es sich bei dem Lungenfunktionsparameter um maximalen Exspirationsfluss MEF, forcierten Exspirationsfluss FEF, forcierte Exspirationsvitalkapazität FVC oder forciertes Exspirationsvolumen FEV, vorzugsweise Einsekundenkapazität FEV1, handelt.

5. Computerlesbares Medium (280), umfassend Kodiermittel, die dazu konfiguriert sind, das Verfahren nach einem der Ansprüche 1 bis 4 zu implementieren.

6. Inhalationssystem (100, 200, 300, 400) zum Verabreichen von Aerosolpartikeln an die Lunge, umfassend:
eine Steuerung (160, 260, 390), die dazu angeordnet ist, Aerosolparameter für das Inhalationssystem auf der Grundlage mindestens eines gemessenen Lungenfunktionsparameters anzupassen, wobei die Steuerung (160, 260, 390) Kodiermittel umfasst, die dazu konfiguriert sind, zu veranlassen, dass das System das Verfahren nach einem der Ansprüche 1 bis 4 implementiert.

7. System nach Anspruch 6, wobei das System einen Leser (275, 392) zum Lesen des computerlesbaren Mediums (280) nach Anspruch 5 oder zum Lesen eines Speichermittels umfasst, auf dem die persönlichen Daten eines Probanden und/oder Aerosolparameter gespeichert sind, und/oder wobei die Steuerung (160, 260, 390) einen Speicher zum Speichern der persönlichen Daten eines Probanden und/oder von Aerosolparametern hat.

8. System nach einem der Ansprüche 6 bis 7, ferner umfassend einen Monitor (420) zum Messen mindestens eines Lungenfunktionsparameters eines Probanden.

9. System nach einem der Ansprüche 6 bis 8, wobei die Aerosolparameter das Inspirationsvolumen von Aerosolpartikeln und/oder aerosolfreien Partikeln, die Inspirationsflussrate und/oder das Timing des Inspirationsflusses von Aerosolpartikeln und/oder aerosolfreien Partikeln umfassen.

10. System nach einem der Ansprüche 6 bis 9, wobei das System eine tragbare, in der Hand gehaltene Inhalationsvorrichtung ist.

## Revendications

1. Procédé pour déterminer l'administration de particules d'aérosol aux poumons, le procédé comprenant :
obtenir des paramètres d'aérosol pour un système d'inhalation (100, 200, 300, 400) en se basant sur au moins un paramètre de fonction pulmonaire mesuré afin d'adapter ainsi l'administration de particules d'aérosol à la fonction pulmonaire du sujet, **caractérisé en ce que**
le paramètre de fonction pulmonaire est une mesure de la fonction expiratoire, et
l'étape consistant à obtenir des paramètres d'aérosol comprend :
sélectionner un coefficient de correction, à l'aide de ladite fonction expiratoire mesurée, parmi une pluralité de coefficients de correction obtenus à partir d'une norme ;
multiplier ladite fonction expiratoire mesurée par ledit coefficient de correction afin d'obtenir une valeur de paramètre de fonction pulmonaire corrigée ;
comparer ladite valeur de paramètre de fonction pulmonaire corrigée à des plages prédéterminées pour des groupes de paramètres d'aérosol préspécifiés, chaque groupe de paramètres d'aérosol comprenant des paramètres d'aérosol prédéfinis ; et
sélectionner un groupe de paramètres d'aérosol préspécifié en se basant sur ladite comparaison.

2. Procédé selon la revendication 1, comprenant en outre l'étape consistant à mesurer au moins un paramètre de fonction pulmonaire d'un sujet.

3. Procédé selon la revendication 1 ou 2, dans lequel ledit coefficient de correction est basé sur la gravité de la maladie pulmonaire du sujet.

4. Procédé selon la revendication 1, 2 ou 3, dans lequel ledit paramètre de fonction pulmonaire est un flux expiratoire maximal MEF, un flux expiratoire forcé FEF, une capacité vitale expiratoire forcée FVC ou un volume expiratoire forcé FEV, de préférence un volume expiratoire forcé par seconde FEV 1.

5. Support lisible par ordinateur (280) comprenant des moyens de codage configurés pour mettre en oeuvre le procédé selon l'une quelconque des revendications 1 à 4.

6. Système d'inhalation (100, 200, 300, 400) servant à l'administration de particules d'aérosol aux poumons comprenant :
un dispositif de commande (160, 260, 390) conçu pour adapter des paramètres d'aérosol pour ledit système d'inhalation en se basant sur au moins un paramètre de fonction pulmonaire mesuré, ledit dispositif de commande (160, 260, 390) comprenant des moyens de codage configurés pour amener le système à mettre en oeuvre le procédé selon l'une quelconque des revendications 1 à 4.

7. Système selon la revendication 6, ledit système comprenant un dispositif de lecture (275, 392) servant à la lecture du support lisible par ordinateur (280) selon la revendication 5 ou à la lecture d'un moyen formant mémoire sur lequel sont mémorisés des données individuelles et/ou des paramètres d'aérosol d'un sujet, et/ou ledit dispositif de commande (160, 260, 390) comprenant une mémoire servant à mémoriser des données individuelles et/ou des paramètres d'aérosol d'un sujet.

8. Système selon la revendication 6 et 7, comprenant en outre un dispositif de surveillance (420) servant à mesurer au moins un paramètre de fonction pulmonaire d'un sujet.

9. Système selon l'une quelconque des revendications 6 à 8, dans lequel lesdits paramètres d'aérosol comprennent le volume inspiratoire de particules d'aérosol et/ou de particules sans aérosol, le débit inspiratoire et/ou le rythme du flux inspiratoire de particules d'aérosol et/ou de particules sans aérosol.

10. Système selon l'une quelconque des revendications 6 à 9, le système étant un dispositif d'inhalation à main portable.
